(19) Europäisches Patentamt
European Patent Office
Office européen des brevets

(11) Numéro de publication: **0 074 894**
**B1**

(12) # FASCICULE DE BREVET EUROPEEN

(45) Date de publication du fascicule du brevet: **02.01.85**

(51) Int. Cl.³: **B 01 D 53/14**, C 01 B 3/52, C 07 C 7/11, C 07 C 9/04

(21) Numéro de dépôt: **82401638.0**

(22) Date de dépôt: **08.09.82**

(54) **Procédé de purification d'un mélange gazeux contenant des composés gazeux indésirables.**

(30) Priorité: **11.09.81 FR 8117233**

(43) Date de publication de la demande: **23.03.83 Bulletin 83/12**

(45) Mention de la délivrance du brevet: **02.01.85 Bulletin 85/1**

(84) Etats contractants désignés: **BE DE FR GB IT NL**

(56) Documents cités: **EP - A - 0 024 746**

(73) Titulaire: **COMPAGNIE FRANCAISE DE RAFFINAGE Société anonyme dite:, 5, rue Michel-Ange, F-75781 Paris Cedex 16 (FR)**
Titulaire: **ANVAR Agence Nationale de Valorisation de la Recherche, 43, rue Caumartin, F-75436 Paris Cédex 09 (FR)**

(72) Inventeur: **Atlani, Martial, 52, rue Bouret, F-75019 Paris (FR)**
Inventeur: **Loutaty, Roben, 29, rue Séry, F-76600 Le Havre (FR)**
Inventeur: **Wakselman, Claude, 18, rue du Clos d'Alençon, F-91120 Villebon sur Yvette (FR)**
Inventeur: **Yacono, Charles, 13 rue de la Mailleraye, F-76600 le Havre (FR)**

(74) Mandataire: **Jolly, Jean-Pierre et al, Cabinet BROT et JOLLY 83, rue d'Amsterdam, F-75008 Paris (FR)**

EP 0 074 894 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne un procédé de purification d'un mélange gazeux contenant des composés gazeux indésirables. Elle concerne plus particulièrement l'élimination de composés indésirables comme les oxydes de carbone, notamment le dioxyde de carbone, les sulfures d'hydrogène et de carbonyle et les alkylthiols.

Au sens de la présente invention, on entend par mélange gazeux à purifier, un mélange gazeux naturel, comme le gaz naturel, ou un mélange gazeux obtenu lors de réactions chimiques, notamment dans l'industrie du raffinage. Ce mélange gazeux peut contenir, notamment, outre au moins un des composés gazeux indésirables cités ci-dessus, des hydrocarbures et/ou de l'hydrogène.

Les procédés actuels de purification de mélanges gazeux font généralement appel à l'une des trois opérations suivantes:

- absorption par un liquide,
- absorption par un solide,
- conversion chimique, essentiellement en présence d'un catalyseur.

La première opération, qui est souvent préférée, pour l'élimination de mélanges gazeux de composés indésirables tels que ceux définis précédemment, met en oeuvre deux types de solvant, séparément ou conjointement:

- des solvants »chimiques«, extrayant les composés indésirables par réaction chimique et les restituant à la suite d'un chauffage, éventuellement accompagné d'une décompression,
- des solvants »physiques«, absorbant les gaz sous pression, par dissolution dans un liquide, et les désorbant par décompression.

Les solvants »physiques« s'avèrent particulièrement intéressants pour des mélanges gazeux portés sous forte pression et contenant un pourcentage élevé de composés indésirables. Dans ces conditions, les solvants »physiques« présentent une capacité d'absorption supérieure à celle des solvants »chimiques«. Cet avantage se traduit par une diminution du taux de solvant à utiliser, une réductions de la taille des équipements et du coût des utilités.

Le but de la présente invention est de proposer des solvants particulièrement adaptés à l'absorption de composés indésirables contenus dans des mélanges gazeux.

A cet effet, l'invention a pour objet un procédé de purification d'un mélange gazeux contenant au moins un composé gazeux indésirable appartenant au groupe constitué par les oxydes de carbone, les sulfures d'hydrogène et de carbonyle et les alkylthiols, ledit procédé comprenant:

a) une première étape d'absorption à l'aide d'un solvant des composés gazeux indésirables, ladite première étape conduisant à:

   - d'une part, un mélange gazeux purifié au moins partiellement,
   - d'autre part, une solution des composés indésirables dans le solvant,

b) une deuxième étape de désorption des composés indésirables de la solution, ladite deuxième étape conduisant:

   - d'une part, à l'obtention d'au moins un composé gazeux indésirable,
   - d'autre part, à l'obtention de solvant qui peut être recyclé à la première étape,

ledit procédé étant caractérisé en ce que le solvant est constitué au moins en partie par une sulfonamide choisie dans le groupe constitué par:

1°) les composés de formule générale:

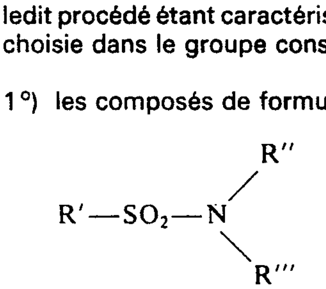

(1)

où R', R'' et R''' peuvent être des restes alkyle ou aryle, saturés ou insaturés, les restes alkyles pouvant être linéaires ou ramifiés et posséder de 1 à 18 atomes de carbone et l'un au moins des restes R'' et R''' pouvant être remplacé par un atome d'hydrogène.

2°) les composés de formule générale

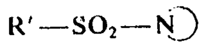 (2)

où R' est un reste tel que le reste R' de la formule (1) et où l'atome d'azote appartient à un cycle composé, outre l'azote, de 2 à 5 atomes de carbone et pouvant contenir, outre l'azote, un hétéroatome, comme l'oxygène par exemple.

3°) les composés de formule générale:

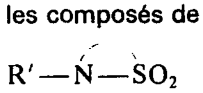 (3)

où R' est un reste tel que le reste R' des formules (1) ou (2) et où le groupement $>N-SO_2$ appartient à un cycle composé, outre dudit groupement, de 2 à 4 atomes de carbone, et pouvant contenir un hétéroatome, comme l'oxygène par exemple.

Parmi les composés (1) définis ci-dessus, on peut citer, par exemple:

- la N-méthylméthane sulfonamide,
- la N-méthyléthane sulfonamide,
- la N-éthylméthane sulfonamide,
- la N-éthyl éthane sulfonamide,
- la N-propylméthane sulfonamide,
- la N-isopropylméthane sulfonamide,
- la N,N-diéthylméthane sulfonamide,
- la N,N-diméthylméthane sulfonamide,
- la N,N-diméthyléthane sulfonamide,
- la N,N-diéthyléthane sulfonamide,
- la N,N-diallylméthane sulfonamide,
- la N,N-méthylallylméthane sulfonamide.

Parmi les composés (2) définis ci-dessus, on peut citer, par exemple:

- la N-éthanesulfonylpyrrolidine,
- la N-éthanesulfonylpipéridine,
- la N-éthane sulfonylmorpholine.

Parmi les composés (3) définis ci-dessus, on peut citer par exemple:

- le N-méthylpropane sultame,
- le N-éthylpropane sultame,
- le N-butylpropane sultame.

Les conditions opératoires de la première étape d'absorption des composés indésirables dépendent de la nature du mélange gazeux à purifier et de la nature du solvant.

A titre d'exemple, pour un mélange gazeux contenant au moins un hydrocarbure léger, comme le méthane, l'éthane ou le propane, et des composés indésirables, comme l'hydrogène sulfuré et le dioxyde de carbone, les conditions opératoires peuvent être les suivantes:

- température comprise entre 5 et 100°C,
- pression comprise entre 4 et 400 bars,
- taux de solvant, c'est-à-dire le rapport

$$\frac{\text{débit molaire du solvant}}{\text{débit molaire du mélange gazeux à purifier}},$$

compris entre 0,05 et 15.

L'étape de désorption des composés indésirables peut être effectuée en une ou plusieurs opérations, par décompression et éventuellement chauffage de la solution obtenue lors de la première étape, avec, éventuellement, un entraînement par un gaz tel que l'azote.

La figure unique, annexée à la présente description, représente, à titre non limitatif, un schéma simplifié d'une unité industrielle mettant en oeuvre le procédé selon l'invention.

Selon ce schéma, on introduit par la ligne 1, dans la partie inférieure d'une colonne d'absorption 2

fonctionnant à contre-courant, un mélange gazeux à purifier contenant du méthane, de l'éthane, du propane et des gaz indésirables que l'on désire éliminer, à savoir de l'hydrogène sulfuré et du dioxyde de carbone.

Il est bien entendu qu'il ne s'agit que d'un exemple, ce gaz pouvant contenir d'autres composés indésirables comme le sulfure de carbonyle ou des alkylthiols.

La colonne 2 peut être par exemple une colonne à garnissage ou à plateaux. Cette colonne peut fonctionner à une température qui peut être comprise entre 5 et 100°C et à une pression qui peut être comprise entre 4 et 400 bars.

Le taux de solvant introduit par la ligne 3, au sommet de la colonne 2, taux qui est le rapport, comme il a été dit précédemment:

$$\frac{\text{débit molaire du solvant}}{\text{débit molaire du mélange gazeux à purifier}},$$

peut être compris entre 0,05 et 15.

On recueille au sommet de la colonne 2, par la ligne 4, le gaz à purifier débarrassé au moins de la majorité de l'hydrogène sulfuré et du dioxyde de carbone qu'il contient. Les dimensions de la colonne et les conditions opératoires de celle-ci sont choisies en fonction du débit et de la pureté du gaz, recueilli par la ligne 4, que l'on désire obtenir.

On recueille au fond de la colonne 2, par la ligne 5, une solution d'hydrogène sulfuré et de dioxyde de carbonne dans le solvant. Cette solution contient également une faible quantité des hydrocarbures contenus dans le gaz à purifier introduit par la ligne 1.

Il faut ensuite régénérer le solvant, c'est-à-dire, dans le cas présent, en extraire l'hydrogène sulfuré et le dioxyde de carbone.

Différents schémas peuvent être envisagés. Sur la figure est représentée en deux temps.

La solution recueillie par la ligne 5 est conduite dans un ensemble constitué par deux étages 7 et 8.

La solution est d'abord introduite dans l'étage 7, où la pression est maintenue à une pression inférieure à celle de la colonne 2, mais supérieure à 1 bar. On recueille au sommet de l'étage 7, par la ligne 9, un mélange d'hydrocarbures, éventuellement un peu d'hydrogène sulfuré et de dioxyde de carbone. Ce mélange, après recompression dans un compresseur 10, est recyclé à la colonne 2, par la ligne 11, après passage dans un réfrigérant 12 destiné à ramener la température du mélange, qui a été réchauffé par suite de la compression, à la température de fonctionnement de la colonne 2.

La solution contenant la plus grande partie de l'hydrogène sulfuré et du dioxyde de carbone est recueillie par la ligne 13 au fond de l'étage 7, et est conduite dans l'étage 8. La pression à l'intérieur de l'étage 8 est inférieure à celle de l'étage 7 et égale ou supérieure à 1 bar.

On recueille au sommet de l'étage 8, par la ligne 14, l'hydrogène sulfuré et le dioxyde de carbone.

On recueille au fond de l'étage 8, par la ligne 15, le solvant débarrassé de l'hydrogène sulfuré et du dioxyde de carbone.

Il est possible d'équiper l'étage 8 d'un dispositif d'entraînement par un gaz de l'hydrogène sulfuré et du dioxyde de carbone. A cet effet, on peut introduire par la ligne 16, dans l'étage 8, de l'azote ou de l'air, qui sont évacués par la ligne 14 en même temps que l'hydrogène sulfuré et le dioxyde de carbone.

La régénération du solvant peut être complétée par un chauffage (non représenté).

Le solvant recueilli par la ligne 15 est recyclé, après passage dans une pompe 17, à la ligne 3.

La ligne 3 peut être équipée d'un échangeur 18, qui peut être un réfrigérant ou un réchauffeur, destiné à amener le solvant à la température de fonctionnement de la colonne 2.

L'installation peut être équipée d'un appoint en solvant (ligne 19) et d'une purge de solvant (ligne 20).

Un lavage à l'eau des effluents gazeux, non représenté, peut limiter d'éventuelles pertes de solvant.

Les exemples qui suivent sont destinés à illustrer l'invention de façon non limitative.

## Exemple 1

Cet exemple est destiné à illustrer l'efficacité de l'extraction de différents gaz indésirables par le procédé selon l'invention à l'aide de différents solvants.

On a mesuré les constantes de HENRY à dilution infinie, à 25°C, de l'éthane, du propane, du butane normal, du dioxyde de carbone, de l'hydrogène sulfuré, du méthylmercaptan et du sulfure de carbonyle.

Ces constantes de HENRY ont été déterminées selon la technique décrite par D. RICHON et H. RENON dans l'article intitulé: »Infinite dilution HENRY'S constants of light hydrocarbons in N-hexadecane, N-octadecane and 2,2,4,4,6,8,8 – hepta méthyl nonane by inert gaz stripping«, publié dans »The Journal of Chemical Engineering Data«, volume 25, Numéro 1, pages 59 et 60 (1980).

La constante de HENRY ci-après désignée par la lettre H, est le rapport

$$H = \frac{\text{pression partielle du gaz}}{\text{fraction molaire du gaz en solution dans le solvant considéré}}.$$

Elle est exprimée en unités de pression.

Ont voit que plus H est élevé, moins le gaz se dissout.

Le Tableau I ci-après donne les constantes de HENRY pour plusieurs gaz considérés et plusieurs solvants.

Les constantes, exprimées en atmosphères, sont désignées par la lettre H, affectées de la formule chimique du gaz considéré.

Tableau 1

| Sulfonamide | Constantes de HENRY | | | | | | |
|---|---|---|---|---|---|---|---|
| | $H_{C_2H_6}$ | $H_{C_3H_8}$ | $H_{C_4H_{10}}$ | $H_{CO_2}$ | $H_{H_2S}$ | $H_{SHCH_3}$ | $H_{COS}$ |
| N,N-diéthyl méthane sulfonamide | 143,5 | 54,4 | 18,9 | 56,1 | 11,8 | 2,2 | 26,1 |
| N-N-diméthyl éthane sulfonamide | 182,0 | 69,1 | 25,7 | 63,3 | 12,3 | 3,1 | 32,8 |
| N-éthyl méthane sulfonamide | 292,6 | 132,1 | 61,4 | 94,5 | 22,8 | 5,5 | 59,3 |
| N,N-méthyl éthyl méthane sulfonamide | 192,2 | 82,3 | 30,3 | 68,4 | 13,5 | 2,5 | 33,8 |
| N-éthyl propane sultame | 244,8 | 93,4 | 38,5 | 65,2 | 12,1 | 2,2 | 26,8 |
| N,N-diallyl méthane sulfonamide | 111,5 | 41,0 | 14,5 | 49,2 | 11,8 | 2,0 | 20,1 |
| N-éthane sulfonyl pyrrolidine | 123,6 | 57,0 | 21,5 | 64,9 | 10,0 | Non mesurées | |

Le Tableau 2 ci-après regroupe certains rapports de constantes de HENRY, prises deux par deux.

Tableau 2

| Sulfonamide | Rapport des constantes de HENRY | | | | | | | | | |
|---|---|---|---|---|---|---|---|---|---|---|
| | $\frac{H_{C_2H_6}}{H_{CO_2}}$ | $\frac{H_{C_2H_6}}{H_{H_2S}}$ | $\frac{H_{C_3H_8}}{H_{H_2S}}$ | $\frac{H_{C_4H_{10}}}{H_{H_2S}}$ | $\frac{H_{CO_2}}{H_{H_2S}}$ | $\frac{H_{C_2H_6}}{H_{SHCH_3}}$ | $\frac{H_{C_3H_8}}{H_{SCH_4}}$ | $\frac{H_{C_4H_{10}}}{H_{SHCH_3}}$ | $\frac{H_{C_2H_6}}{H_{COS}}$ | $\frac{H_{C_3H_8}}{H_{COS}}$ |
| N,N-diéthyl méthane sulfonamide | 2,56 | 12,2 | 4,61 | 1,60 | 4,75 | 65,2 | 24,7 | 8,59 | 5,50 | 2,08 |
| N,N-diméthyl éthane sulfonamide | 2,87 | 14,8 | 5,62 | 2,09 | 5,15 | 58,7 | 22,3 | 8,29 | 5,55 | 2,11 |
| N-éthyl méthane sulfonamide | 3,10 | 12,8 | 5,79 | 2,69 | 4,14 | 53,2 | 84,0 | 11,2 | 4,93 | 2,23 |
| N,N-méthyl éthyl méthane sulfonamide | 2,81 | 14,2 | 6,10 | 2,24 | 5,07 | 76,9 | 32,9 | 12,1 | 5,69 | 2,43 |

Suite

| Sulfonamide | Rapport des constantes de HENRY | | | | | | | | | |
|---|---|---|---|---|---|---|---|---|---|---|
| | $\frac{H_{C_2H_6}}{H_{CO_2}}$ | $\frac{H_{C_2H_6}}{H_{H_2S}}$ | $\frac{H_{C_3H_8}}{H_{H_2S}}$ | $\frac{H_{C_4H_{10}}}{H_{H_2S}}$ | $\frac{H_{CO_2}}{H_{H_2S}}$ | $\frac{H_{C_2H_6}}{H_{SHCH_3}}$ | $\frac{H_{C_3H_8}}{H_{SCH_4}}$ | $\frac{H_{C_4H_{10}}}{H_{SHCH_3}}$ | $\frac{H_{C_2H_6}}{H_{COS}}$ | $\frac{H_{C_3H_8}}{H_{COS}}$ |
| N-éthyl propane sultame | 3,75 | 20,2 | 7,72 | 3,18 | 5,39 | 111,3 | 42,5 | 17,5 | 9,13 | 3,48 |
| N,N-diallyl méthane sulfon-amide | 2,27 | 9,45 | 3,47 | 1,23 | 4,17 | 55,8 | 20,5 | 7,25 | 5,55 | 2,04 |
| N-éthane sulfonyl pyrrolidine | 1,90 | 12,4 | 5,70 | 2,15 | 6,49 | | | | | |

Plus le rapport est élevé, meilleure est la séparation.

Le Tableau 2 montre la possibilité des séparations suivantes par le procédé selon l'invention:

- éthane — dioxyde de carbone,
- éthane — hydrogène sulfuré,
- propane — hydrogène sulfuré,
- butane normal — hydrogène sulfuré,
- dioxyde de carbone — hydrogène sulfuré,
- éthane — méthyl thiol,
- propane — méthyl thiol,
- butane normal — méthyl thiol,
- éthane — sulfure de carbonyle,
- propane — sulfure de carbonyle,

Le procédé selon l'invention permet donc la séparation efficace de gaz indésirables, tels que le dioxyde de carbone, l'hydrogène sulfuré, le méthyl thiol et le sulfure de carbonyle, de gaz en contenant. Il peut servir à l'absorption sélective de l'hydrogène sulfuré dans le cas où le mélange gazeux contient simultanément le dioxyde de carbone et l'hydrogène sulfuré.

## Exemple 2

Cet exemple concerne l'élimination de l'hydrogène sulfuré et du dioxyde de carbone d'un mélange gazeux à l'aide de la N,N-diméthyl éthane sulfonamide.

Le mélange gazeux, un gaz naturel, a la composition molaire suivante:

- méthane: 73%,
- éthane: 1,5%,
- propane: 0,5%,
- hydrogène sulfuré: 15%,
- dioxyde de carbone: 10%,

Le mélange gazeux est introduit dans le fond d'une colonne d'absorption ayant 10 étages théoriques, alimentée à son sommet par de la N-N-diméthyléthane sulfonamide.

La température et la pression à l'intérieur de la colonne sont respectivement 25 °C et 100 bars. Le taux de solvant est de 0,4.

On recueille au sommet de la colonne le gaz purifié, dont la composition molaire est la suivante:

- méthane: 93,1%,
- éthane: 1,6%,
- propane: 0,4%,
- hydrogène sulfuré: $<$ 2 p.p.m.,
- dioxyde de carbone 4,9%

La solution recueillie au fond de la colonne a la composition suivante:

- méthane: 4,6%,
- éthane: 0,5%,
- propane: 0,3%,
- hydrogène sulfuré: 23,1%,
- dioxyde de carbone: 9,7%,
- N,N-diméthyléthane sulfonamide: 61,8%.

Cet exemple montre bien l'efficacité du procédé selon l'invention pour éliminer l'hydrogène sulfuré et le dioxyde de carbone du gaz naturel.

**Revendications**

1. Procédé de purification d'un mélange gazeux composé notamment d'hydrocarbures et /ou d'hydrogène et contenant en outre au moins un composé gazeux indésirable appartenant au groupe constitué par les oxydes de carbone, les sufures d'hydrogène et de carbonyle et les alkylthiols, ledit procédé comprenant:

a) une première étape d'absorption des composés gazeux indésirables dans un solvant, ladite première étape conduisant:

- d'une part, à un mélange gazeux purifié au moins partiellement,
- d'autre part, à une solution des composés indésirables dans le solvant.

b) une deuxième étape de désorption des composés indésirables de la solution, ladite deuxième étape conduisant:

- d'une part, à l'obtention d'au moins un composé gazeux indésirable,
- d'autre part, à l'obtention de solvant qui peut être recyclé à la première étape,

ledit procédé étant caractérisé en ce que le solvant est constitué au moins en partie par une sulfonamide choisie dans le groupe constitué par:

1/ les composés de formule générale:

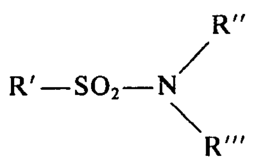

(1)

où R', R″ et R‴ peuvent être des restes alkyle ou aryle, saturés ou insaturés, les restes alkyles pouvant être linéaires ou ramifiés, et posséder de 1 à 18 atomes de carbones et l'un au moins des restes R″ et R‴ pouvant être remplacé par un atome d'hydrogène.

2/ les composés de formule générale:

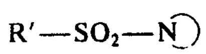

(2)

où R' est un reste tel que le reste R' de la formule (1) et où l'atome d'azote appartient à un cycle composé, outre l'azote, de 2 à 5 atomes de carbone, et pouvant contenir outre l'azote, un hétéroatome, comme l'oxygène par exemple.

3/ les composés de formule générale:

$$R'-N-SO_2 \quad (3)$$

où R' est un reste tel que le reste R' des formule (1) ou (2) et où le groupement $>N-SO_2-$ appartient à un cycle composé, outre dudit groupement, de 2 à 4 atomes de carbone et pouvant contenir un hétéroatome comme l'oxygène par exemple.

2. Procédé selon la revendication 1, caractérisé en ce que la sulfonamide est choisie dans le groupe constitué par:

- la N-méthylméthane sulfonamide,
- la N-méthyléthane sulfonamide,
- la N-méthylméthane sulfonamide,
- la N-éthyl éthane sulfonamide,
- la N-propylméthane sulfonamide,
- la N-isopropylméthane sulfonamide,
- la N,N-diéthylméthane sulfonamide,
- la N,N-diméthylméthane sulfonamide,
- la N,N-diméthyléthane sulfonamide,
- la N,N-diéthyléthane sulfonamide,
- la N,N-diallylméthane sulfonamide,
- la N,N-méthylallylméthane sulfonamide,
- la N-éthanesulfonylpyrrolidine,
- la N-éthanesulfonylpipéridine,
- la N-éthane sulfonylmorpholine,
- le N-méthylpropane sultame,
- le N-éthylpropane sultame,
- le N-butylpropane sultame.

3. Procédé selon l'une des revendications 1 et 2, caractérisé en ce que l'étape d'absorption est effectuée à une température comprise entre 5 et 100°C.

4. Procédé selon l'une des revendications 1 à 3, caractérisé en ce que l'étape d'absorption est effectuée à une pression comprise entre 4 et 400 bars.

5. Procédé selon l'une des revendications 1 à 4, caractérisé en ce que le taux de solvant, c'est à dire le rapport du débit molaire de solvant au débit molaire du mélange gazeux à purifier est compris entre 0,05 et 5.

**Patentansprüche**

Verfahren zur Reinigung eines gasförmigen Gemisches, welches sich insbesondere aus Kohlenwasserstoffen und/oder Wasserstoff zusammensetzt und außerdem mindestens eine unerwünschte gasförmige Verbindung aus der Gruppe bestehend aus den Kohlenoxyden, Schwefelwasserstoff, den Carbonylsulfiden und den Alkylmercaptanen enthält, mit

a) einem ersten Schritt zur Absorption der unerwünschten gasförmigen Verbindungen in einem Lösungsmittel, welcher einerseits zu einem wenigstens teilweise gereinigten gasförmigen Gemisch und andererseits zu einer Lösung der unerwünschten Verbindungen im Lösungsmittel führt, und
b) einem zweiten Schritt zur Desorption der unerwünschten Verbindungen aus der Lösung, welcher einerseits mindestens eine unerwünschte gasförmige Verbindung und andererseits Lösungsmittel erhalten läßt, welches zum ersten Schritt rückgeführt werden kann,

dadurch gekennzeichnet, daß das Lösungsmittel wenigstens teilweise aus einem Sulfonamid besteht, welches aus der Gruppe ausgewählt wird, die besteht aus:

1. den Verbindungen der allgemeinen Formel

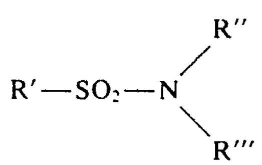

(1)

wobei R', R'' und R''' gesättigte oder ungesättigte Alkyl- oder Arylreste darstellen können, welche Alkylreste linear oder verzweigt sein und 1 bis 18 C-Atome aufweisen können, und wobei wenigstens einer der Reste R'' und R''' durch ein Wasserstoffatom ersetzt sein kann,

2. den Verbindungen der allgemeinen Formel

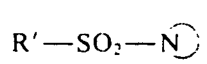

(2)

wobei es sich beim Rest R' um denjenigen der Formel (1) handelt und das Stickstoffatom zu einem außer dem Stickstoffatom aus 2 bis 5 C-Atomen bestehenden Ring gehört, welcher außer dem Stickstoffatom ein weiteres Heteroatom, wie beispielsweise ein Sauerstoffatom, enthalten kann, und

3. den Verbindungen der allgemeinen Formel

$$R'—N—SO_2 \qquad (3)$$

wobei es sich beim Rest R' um denjenigen der Formel (1) oder (2) handelt und die (=N−$SO_2$−)-Gruppe zu einem außer dieser Gruppe aus 2 bis 4 C-Atomen bestehenden Ring gehört, welcher ein weiteres Heteroatom, wie beispielsweise ein Sauerstoffatom enthalten kann.

2. Verfahren nach Anspruch 1, dadurch gekennzeichnet, daß das Sulfonamid aus der Gruppe ausgewählt wird, die besteht aus:

N-Methyl-methan-sulfonamid
N-Methyl-äthan-sulfonamid
N-Äthyl-methan-sulfonamid
N-Äthyl-äthan-sulfonamid
N-Propyl-methan-sulfonamid
N-Isopropyl-methan-sulfonamid
N,N-Diäthyl-methan-sulfonamid
N,N-Dimethyl-methan-sulfonamid
N,N-Dimethyl-äthan-sulfonamid
N,N-Diäthyl-äthan-sulfonamid
N,N-Diallyl-methan-sulfonamid
N,N-Methyl-allyl-methan-sulfonamid
N-Äthan-sulfonyl-pyrrolidin
N-Äthan-sulfonyl-piperidin
N-Äthan-sulfonyl-morpholin
N-Methyl-propan-sultam
N-Äthyl-propan-sultam
N-Butyl-propan-sultam

3. Verfahren nach Anspruch 1 oder 2, dadurch gekennzeichnet, daß der Absorptionsschritt bei einer Temperatur zwischen 5 und 100°C durchgeführt wird.

4. Verfahren nach einem der Ansprüche 1 bis 3, dadurch gekennzeichnet, daß der Absorptionsschritt bei einem Druck zwischen 4 und 400 bar durchgeführt wird.

5. Verfahren nach einem der Ansprüche 1 bis 4, dadurch gekennzeichnet, daß das Lösungsmittelverhältnis, d. h. das Verhältnis des molaren Lösungsmitteldurchsatzes zum molaren Durchsatz des zu reinigenden gasförmigen Gemisches, zwischen 0,05 und 5 liegt.

## Claims

1. A process for purifying a gaseous mixture formed more especially from hydrocarbons and/or hydrogen and further containing at least one undesirable gaseous compound belonging to the group formed by the carbon oxides, the hydrogen and carbonyl sulphides and the alkylthiols, said process comprising:

a) a first step for absorbing the undesirable gaseous compounds in a solvent, said first step leading:

- on the one hand, to a gaseous mixture at least partially purified,
- on the other hand, to a solution of the undesirable compounds in the solvent,

b) a second step of desorbing the undesirable compounds from the solution, said second step leading:

- on the one hand, to obtaining at least one undesirable gaseous compound
- on the other hand, to obtaining a solvent which may be recycled to the first step,

said process being characterized in that the solvent is formed at least partly by a sulfonamide chosen from the group formed by:

1. the compounds of the general formula:

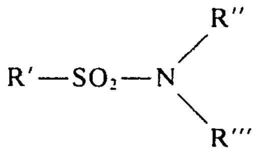

(1)

where R', R'' and R''' may be saturated or unsaturated alkyl or aryl remainders, the alkyl remainders being either linear or ramified and having 1 to 18 carbon atoms, and one at least of the R'' and R''' remainders being able to be replaced by a hydrogen atom.

2. The compounds of the general formula:

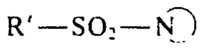

where R' is a remainder such as the remainder R' of formula (1) and where the nitrogen atom belongs to a cycle formed, in addition to nitrogen, of two to five carbon atoms and possibly further containing nitrogen, a heteroatom, such as oxygen for example,

3. the compounds of the general formula:

$$R'—N—SO_2 \quad (3)$$

where R' is a remainder such as the remainder R' of formulas (1) or (2) and where the $>N-SO_2$- group belongs to a cycle formed, in addition to said group, from two to four carbon atoms and possibly containing a heteroatom such as oxygen for example.

2. The process according to claim 1, characterized in that the sulfonamide is chosen from the group formed by:

- N-methylmetane sulfonamide,
- N-methylethane sulfonamide,
- N-methylmethane sulfonamide,
- N-ethyl ethane sulfonamide,

N-propylmethane sulfonamide,
N-isopropylmethane sulfonamide,
N,N-diethylmethane sulfonamide,
N,N-dimethylmethane sulfonamide,
N,N-dimethylethane sulfonamide,
N,N-diethylethane sulfonamide,
N,N-diallylmethane sulfonamide,
N,N-methylallylmethane sulfonamide,
N-ethanesulfonylpyrrolidine,
— N-ethanesulfonylpiperidine,
N-ethane sulfonylmorpholine,
N-methylpropane sultame,
N-ethylpropane sultame,
N-butylpropane sultame.

3. The process according to one of claims 1 and 2, characterized in that the absorption step is carried out at a temperature between 5 and 100°C.

4. The process according to one of claims 1 to 3, characterized in that the absorption step is carried out at a pressure between 4 and 400 bars.

5. The process according to one of claims 1 to 4, characterized in that the proportion of solvent, i.e. the ratio of the solvent molar flow to the molar flow of the gaseous to be purified, is between 0.05 and 5.

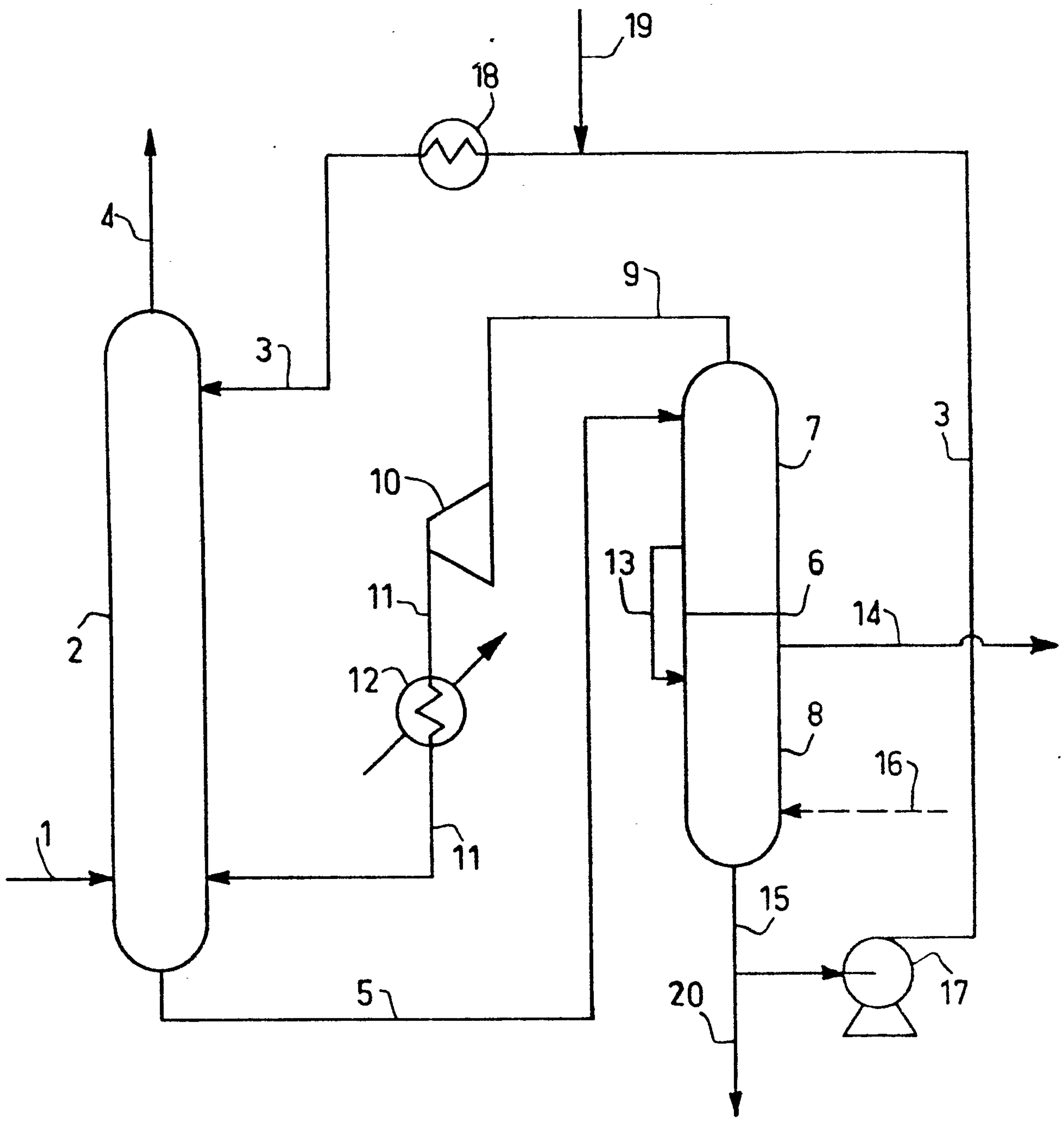
19
18
4
9
3
7
3
10
13
6
11
14
2
12
8
16
1
11
15
5
20
17